# EUROPÄISCHE PATENTSCHRIFT

(11) **EP 1 690 855 B1**
(45) Veröffentlichungstag und Bekanntmachung des Hinweises auf die Patenterteilung: **22.01.2014**
(21) Anmeldenummer: 06111503.6
(22) Anmeldetag: 21.11.2001
(51) Int. Cl.: A61Q 17/04, C07D 235/20, C07D 235/18, A61K 8/49

(54) **Mischung umfassend Phenylen-bis-benzimidazol-tetrasulfonsäure-dinatriumsalz**
Composition comprising phenylene-bis-benzimidazol-tetrasulfonic acid disodium salt
Composition comprenant le sel disodique de l'acide tétrasulfonique du phénylène-bis-benzimidazole.

(30) Priorität: 29.11.2000 DE 10059254
(43) Veröffentlichungstag der Anmeldung: 16.08.2006
(62) Teilanmeldung aus: 01990428.3
(73) Patentinhaber: Symrise AG, 37603 Holzminden (DE)
(72) Erfinder: BERTRAM, Ralf, 37603, Holzminden (DE); HILLERS, Stephan, 20099 Hamburg (DE); KOCH, Oskar, 37079, Göttingen (DE); ERFURT, Harry, 37170, Uslar (DE); REINDERS, Gerald, 37671, Höxter (DE)
(74) Vertreter: Eisenführ Speiser

(56) Entgegenhaltungen:
- EP-A- 1 027 882
- EP-A2- 0 868 904
- DE-A- 4 409 689
- DE-A1- 19 846 772
- US-A- 2 463 264
- US-A- 3 536 730
- US-A- 5 473 079

## Beschreibung

Die vorliegende Erfindung betrifft eine Mischung umfassend Phenylen-bis-benzimidazol-tetrasulfonsäure-dinatriumsalz, das frei von typischen verfärbenden Verunreinigungen ist.

Die Herstellung des Phenylen-bis-benzimidazol-tetrasulfonsäure-dinatriumsalzes ist an sich bekannt (DE A 440 96 89). Das Verfahren kann durch das folgende Formelschema erläutert werden:

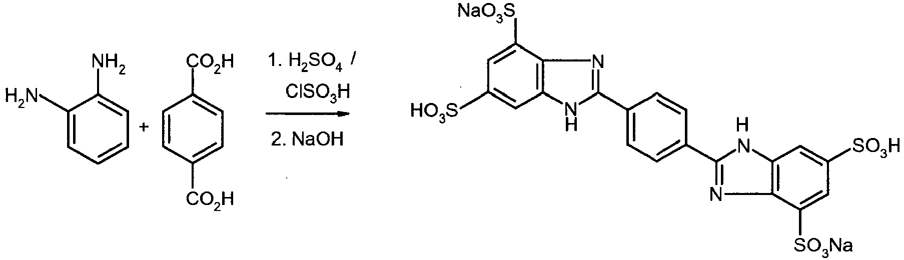

Phenylen-bis-benzimidazol-tetrasulfonsäure-dinatriumsalz wird danach bei einer Temperatur von etwa 120°C bei einer Reaktionszeit von einer Stunde hergestellt. Anschließend erfolgt Hydrolyse in Eiswasser, wobei Phenylen-bis-benzimidazol-tetra-sulfonsäure-dinatriumsalz auskristallisiert; das Kristallisat wird abfiltriert. Dann wird das noch feuchte Kristallisat in einer Lauge, wie Natronlauge, aufgenommen und gelöst, dann mit Aktiv-Kohle versetzt und erhitzt. Man filtriert die Aktiv-Kohle ab und fällt das Phenylen-bis-benzimidazol-tetrasulfonsäure-dinatriumsalz durch Zugabe von Schwefelsäure wieder aus. Nach Trocknung liegt pulverförmiges Phenylen-bis-benzimidazol-tetrasulfonsäure-dinatriumsalz mit 99 Gew.-% Reinheit vor.

Bei den anwendungstechnischen Prüfungen des so hergestellten Phenylen-bis-benzimidazol-tetrasulfonsäure-dinatriumsalz zeigt sich, dass es bei Einsatz dieses nach o.a. Verfahren hergestellten Produktes zu Verfärbungen in den Formulierungen kam, die nicht akzeptabel sind.

Ursache für die Verfärbungen sind u.a. Diphenylamin, 3,4-Diamino-benzolsulfonsäure und 2-(4'-Carboxy-phenyl)-benzimidazol-6-sulfonsäure.

Aufgabe der vorliegenden Erfindung ist die Herstellung von Mischungen, umfassend Phenylen-bis-benzimidazol-tetrasulfonsäure-dinatriumsalz in hoher Reinheit, das insbesondere keine sich verfärbende Anteile enthält.

Gelöst wird diese Aufgabe durch den Gegenstand des Anspruches 1 und die Gegenstände der von Anspruch 1 abhängigen Ansprüche.

Es wurde ein Verfahren zur Herstellung von Phenylen-bis-benzimidazol-tetrasulfonsäure-dinatriumsalz durch Umsetzung von o-Phenylendiamin mit Terephthalsäure und Chlorsulfonsäure in Gegenwart von starken Säuren gefunden, das dadurch gekennzeichnet ist, dass die Reaktionszeit 10 bis 15 Stunden beträgt.

Nach diesem Verfahren erhält man Phenylen-bis-benzimidazol-tetrasulfonsäure-dinatriumsalz in einer Reinheit von über 98 Gew.-%. Nebenprodukte sind lediglich

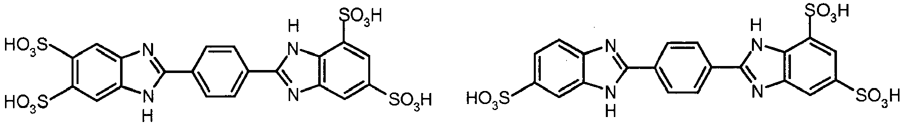

Die Nebenprodukte sind unbedenklich und rufen keine Verfärbung des Phenylen-bis-benzimidazol-tetrasulfonsäure-dinatriumsalz hervor.

Die bevorzugte Reaktionszeit für das Verfahren beträgt 11 bis 12 Stunden.

In einer bevorzugten Ausführungsform des Verfahrens wird die bei der Reaktion bzw. nach Hydrolyse erhaltene Phenylen-bis-benzimidazol-tetrasulfonsäure in einem ersten Schritt in Wasser gelöst und mit Aktiv-Kohle versetzt, die danach abgetrennt wird und wobei aus dem Filtrat das Phenylen-bis-benzimidazol-tetrasulfonsäure-dinatriumsalz mit Natriumchlorid ausgefällt und abgetrennt wird, das in einem zweiten Schritt erneut in Wasser und Natronlauge gelöst und nochmals mit Aktiv-Kohle versetzt wird, die danach wieder abgetrennt wird, wobei aus dem Filtrat durch Ansäuern reines Phenylen-bis-benzimidazol-tetrasulfonsäure-dinatriumsalz ausfällt, das gegebenenfalls anschließend noch gereinigt wird.

Die bei der Reaktion bzw. nach Hydrolyse erhaltene Phenylen-bis-benzimidazol-tetrasulfonsäure wird im ersten Schritt in Wasser im Temperaturbereich von 40 bis 80°C, bevorzugt 45 bis 55°C gelöst.

Vorzugsweise stellt man hierbei eine 1 bis 30 Gew.-%, im besonderen bevorzugt 5 bis 7 Gew.-% Lösung von Phenylen-bis-benzimidazol-tetrasulfonsäure her.

Aktivkohle für das Verfahren (erster und zweiter Schritt) können alle handelsüblichen Typen sein.

Zum Ausfällen des Natriumsalzes gibt man im allgemeinen eine 1 bis 15, bevorzugt 3 bis 10 äquimolare Menge Natriumchlorid hinzu.

Das im ersten Schritt erhaltene Phenylen-bis-benzimidazol-tetrasulfonsäure-dinatriumsalz wird im zweiten Schritt in Wasser im Temperaturbereich von 30 bis 80°C, bevorzugt von 45 bis 50°C gelöst.

Vorzugsweise stellt man hierbei eine 5 bis 25 Gew.-%, im besonderen bevorzugt 15 bis 20 Gew.-% Lösung von Phenylen-bis-benzimidazol-tetrasulfonsäure-dinatriumsalz her.

Das im ersten Schritt erhaltene Phenylen-bis-benzimidazol-tetrasulfonsäure-dinatriumsalz wird gelöst und nach Versetzen und Abtrennen der Aktivkohle durch Ansäuern auf etwa pH 3 ausgefällt. Das Ansäuern erfolgt vorzugsweise mit Salzsäure. Auch hierbei fällt überraschenderweise das Dinatriumsalz aus.

Das im zweiten Schritt erhaltene Phenylen-bis-benzimidazol-tetrasulfonsäure-dinatriumsalz kann in einer bevorzugten Ausführungsform noch mit Phosphorsäure gewaschen werden.

Das Verfahren kann beispielsweise wie folgt durchgeführt werden:
Man legt Schwefelsäure, Terephthalsäure sowie o-Phenylendiamin vor und erhitzt das Gemisch auf beispielsweise 110°C unter Stickstoffatmosphäre. Danach wird innerhalb von 4 h Chlorsulfonsäure bei einer Temperatur zwischen 110 und 120°C zudosiert und anschließend noch 12 h weitergerührt. Die nach Hydrolyse erhaltene Phenylen-bis-benzimidazol-tetrasulfonsäure wird bei 35-40°C in Wasser gelöst, mit Aktiv-Kohle versetzt und etwa 30 min bei gleicher Temperatur gerührt. Die Aktiv-Kohle wird abfiltriert und das Filtrat mit Natriumchlorid versetzt und innerhalb von etwa 2 Stunden zur Ausfällung langsam auf Raumtemperatur unter Rühren abgekühlt. Dann wird das so erhaltene Phenylen-bis-benzimidazol-tetrasulfonsäure-dinatriumsalz abfiltriert und mit 5-proz. Kochsalzlösung nachwaschen.

Das so erhaltene Phenylen-bis-benzimidazol-tetrasulfonsäure-dinatriumsalz wird erneut in Wasser eintragen und zur Lösung 45-proz. Natronlauge zugegeben bis auf pH 5, nochmals Aktiv-Kohle zugesetzt. Die Mischung wird auf 55°C gehalten und etwa 2 Stunden gerührt. Die Aktiv-Kohle wird abgetrennt und das Filtrat zur erneuten Ausfällung des Phenylen-bis-benzimidazol-tetrasulfonsäure-dinatriumsalz mit reiner Salzsäure bis pH 3 angesäuert. Es wird ca. 2 Stunden gerührt und auf Raumtemperatur abgekühlt. Das Phenylen-bis-benzimidazol-tetrasulfonsäure-dinatriumsalz wird in an sich bekannter Weise, z.B. durch Filtration, abgetrennt.

Das so erhaltene Phenylen-bis-benzimidazol-tetrasulfonsäure-dinatriumsalz kann dann noch mit einer 2 Gew.-% wässrigen Natriumchloridlösung, die mit geringen Mengen Phosphorsäure auf pH 3 gestellt ist, gewaschen werden; anschließend wird beispielsweise bei 140°C und 2 mbar getrocknet.

Mit Hilfe des beschriebenen Verfahrens erhält man ein im Spurenbereich analytisch einwandfreies Produkt, das für den Einsatz in kosmetischen Formulierungen bestens geeignet ist und die eingangs aufgeführten Nachteile der Verfärbungen nicht besitzt. Überraschenderweise wird die Anwesenheit von problematischen Spurenkomponenten verhindert.

Phenylen-bis-benzimidazol-tetrasulfonsäure-dinatriumsalz kann als UV-A-Filter in kosmetischen Produkten verwendet werden.

### Beispiel:

1.703 g Schwefelsäure, 96 % werden vorgelegt und 232 g Terephthalsäure eingetragen, anschließend gibt man 302 g o-Phenylendiamin nachdem mit Stickstoff gespült wurde portionsweise hinzu, wobei die Temperatur auf 97°C ansteigt. Die Temperatur wird danach dann auf 110°C erhöht. Dann werden 2.200 g Chlorsulfonsäure innerhalb 4 h dosiert, Temperatur dabei zwischen 110-120°C gehalten, nach Dosierung noch 12 h bei genannter Temperatur rühren.

Der Reaktorinhalt wird auf Raumtemperatur abgekühlt und 4.000 g Wasser von 5°C innerhalb 4 h dosiert, wobei Temperatur auf ca. 47°C steigt, anschl. wird noch 2 h nachgerührt und dann filtriert. Man erhält 1.200 g feuchte Phenylen-bis-benzimidazol-tetrasulfonsäure.

Dieser Presskuchen wird in 11.000 g Wasser von 40°C eingetragen und gelöst, dann erfolgt Zugabe von 30 g A-Kohle. Das Gemisch wird 30 min unter Stickstoff gerührt und dann filtriert. Das Filtrat wird bei 50°C mit 600 g Kochsalz versetzt und 2 h bei dieser Temperatur gerührt, wobei gegen Ende der Rührzeit auf 25°C abgesenkt wird; dann erfolgt Filtration. Das erhaltene Produkt wird mit 2.800 g 5 Gew.-% Kochsalzlösung gewaschen. Man erhält 1.362 g Phenylen-bis-benzimidazol-tetrasulfonsäure-dinatriumsalz.

Das so erhaltene Phenylen-bis-benzimidazol-tetrasulfonsäure-dinatriumsalz wird in 4.500 g Wasser von 50°C eingetragen und suspendiert (pH 2,4); dann erfolgt bei 50°C Zugabe von 168 g 45-proz. Natronlauge zur Einstellung auf pH 5 und Lösung des Produkts, dann werden 30 g A-Kohle zugesetzt, Ansatz auf 55°C erwärmt und 2 h nachgerührt und filtriert, Filtrat wird mit 162 g Salzsäure, rein versetzt, dabei wird die Temperatur bei 50°C gehalten sowie ein pH von 3 eingestellt, man rührt 2 h unter Stickstoff und kühlt dabei auf 25°C ab; dann erfolgt Filtration, der Filterkuchen wird anschließend mit 4.000 g 2-proz. Kochsalzlösung, die mit wenig Phosphorsäure auf pH 3 eingestellt ist, gewaschen, man erhält 1.155 g des so gereinigten Phenylen-bis-benzimidazol-tetrasulfonsäure-dinatriumsalz.

Das so gereinigte Phenylen-bis-benzimidazol-tetrasulfonsäure-dinatriumsalz wird dann 10 h bei 140°C und 2 mbar getrocknet. Man erhält 700 g Endprodukt (Restfeuchte 2 %). Das so erhaltene Phenylen-bis-benzimidazol-tetrasulfonsäure-dinatriumsalz hat eine Reinheit von 98 Gew.-% und enthält als Nebenprodukte lediglich

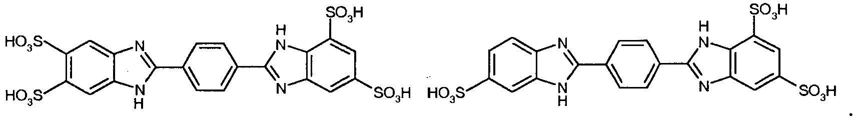

Die Nebenprodukte sind unbedenklich und rufen keine Verfärbung des Phenylen-bis-benzimidazol-tetrasulfonsäure-dinatriumsalz hervor.

## Patentansprüche

1. Mischung, umfassend
Phenylen-bis-benzimidazol-tetrasulfonsäure-dinatriumsalz
wobei die Maßgabe gilt, dass
die Mischung keinen sie und/oder das Phenylen-bis-benzimidazol-tetrasulfonsäure-dinatriumsalz verfärbenden Anteil an Diphenylamin, 3,4-Diamino-benzolsulfonsäure und 2-(4'-Carboxy-phenyl)-benzimidazol-6-sulfonsäure enthält.

2. Mischung nach Anspruch 1, wobei der Anteil an Phenylen-bis-benzimidazol-tetrasulfonsäure-dinatriumsalz größer ist als 98 Gew.-%, bezogen auf die Gesamtmasse der Mischung.

3. Mischung nach einem der vorangehenden Ansprüche, umfassend 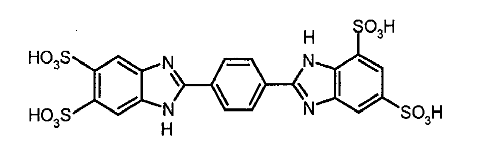 und/oder

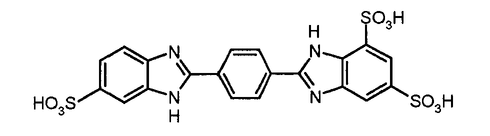

4. Mischung nach Anspruch 3, bestehend aus
(a) Phenylen-bis-benzimidazol-tetrasulfonsäure-dinatriumsalz
(b) 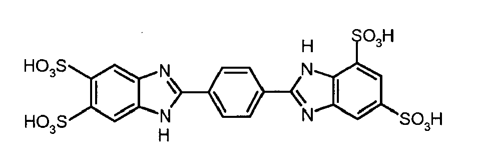 und
(c)

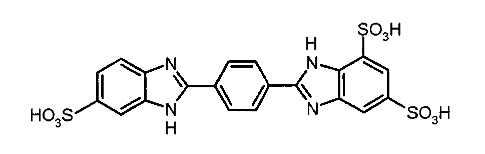

5. Mischung nach einem der vorangehenden Ansprüche, herstellbar durch Umsetzung von o-Phenylen-diamin mit Terephtalsäure und Chlorsulfonsäure in Gegenwart von starken Säuren, **dadurch gekennzeichnet, dass** die Reaktionszeit 10 bis 15 Stunden beträgt, die bei der Reaktion erhaltene Phenylen-bis-benzimidazol-tetrasulfonsäure, in einem ersten Schritt in Wasser gelöst und mit Aktiv-Kohle versetzt wird, die danach abgetrennt wird und wobei aus dem Filtrat das Phenylen-bis-benzimidazol-tetrasulfunsäure-dinatriumsalz durch Zugabe von Natriumchlorid ausgefällt und abtrennt wird, das in einem zweiten Schritt erneut in Wasser und Natronlauge gelöst und nochmals mit Aktiv-Kohle versetzt wird, die danach wieder abgetrennt wird, wobei aus dem Filtrat durch Ansäuern reines Phenylen-bis-benzimidazol-tetrasulfonsäure-dinatriumsalz ausfällt, das gegebenenfalls anschließend noch gereinigt wird.

6. Kosmetische Formulierung, umfassend eine Mischung nach einem der vorangehenden Ansprüche.

## Claims

1. Mixture, comprising
phenylene-bis-benzimidazole-tetrasulfonic acid di-sodium salt
with the proviso that
the mixture does not contain a portion of diphenyl amine, 2,4-diamino benzenesulfonic acid and 2-(4'-carboxy-phenyl)-benzimidazole-6-sulfonic acid, which discolors it and/or the phenylene-bis-benzimidazole-tetrasulfonic acid di-sodium salt.

2. Mixture according to claim 1, wherein the portion of phenylene-bis-benzimidazole-tetrasulfonic acid di-sodium salt is larger than 98 wt.-%, with respect to the total mass of the mixture.

3. Mixture according to one of the preceding claims, comprising 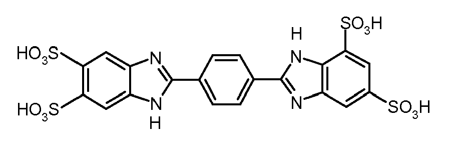 and/or

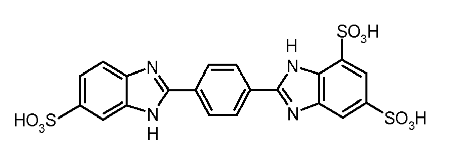

4. Mixture according to claim 3, consisting of
(a) phenylene-bis-benzimidazole-tetrasulfonic acid di-sodium salt
(b) 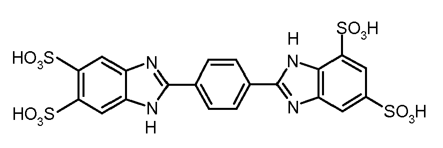 and
(c)

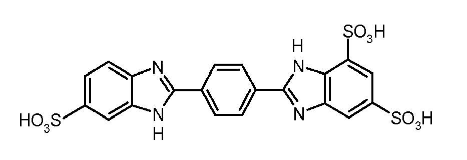

5. Mixture according to one of the preceding claims, producible by reacting o-phenylenediamine with terephtalic acid and chlorosulfuric acid in the presence of strong acids, **characterized in that** the reaction time is 10 to 15 hours, the phenylene-bis-benzimidazole-tetrasulfonic acid obtained in the reaction is in a first step dissolved in water and mixed with activated charcoal, which is subsequently separated off and wherein the phenylene-bis-benzimidazole-tetrasulfonic acid di-sodium salt is precipitated and separated from the filtrate by addition of sodium chloride, which is in a second step again dissolved in water and sodium hydroxide solution and again mixed with activated charcoal, which is subsequently again separated off, wherein pure phenylene-bis-benzimidazole-tetrasulfonic acid di-sodium salt precipitates from the filtrate when acidified, which is subsequently purified if applicable.

6. Cosmetic formulation, comprising a mixture according to one of the preceding claims.

## Revendications

1. Mélange comprenant du sel disodique de l'acide phénylène-bis-benzimidazol-tétrasulfonique, à condition que ledit mélange ne contienne pas de part de diphé-nylamine, d'acide 3,4-diamino-benzènesulfonique et d'acide 2-(4'-carboxy-phényl)-benzimidazol-6-sulfonique qui change la couleur de celui-ci et/ou du sel disodique de l'acide phénylène-bis-benzimidazol-tétrasulfonique.

2. Mélange selon la revendication 1, dans lequel la part de sel disodique de l'acide phénylène-bis-benzimidazol-tétrasulfonique est supérieure à 98 % en poids par rapport à la masse totale du mélange.

3. Mélange selon l'une quelconque des revendications précédentes, comprenant 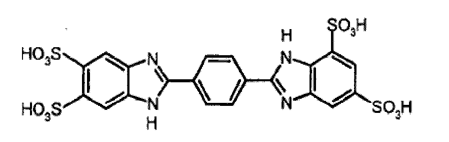 et/ou

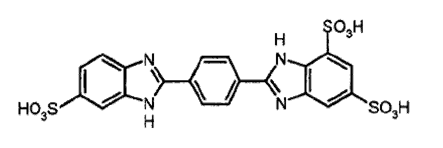

4. Mélange selon la revendication 3, se composant de
(a) sel disodique de l'acide phénylène-bis-benzimidazol-tétrasulfonique
(b) 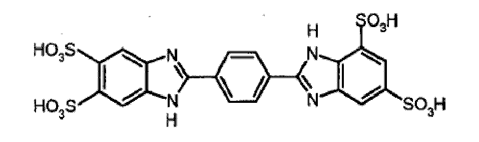 et
(c)

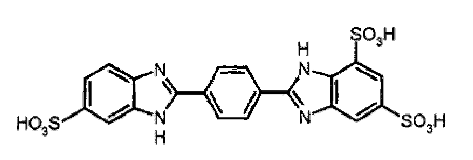

5. Mélange selon l'une quelconque des revendications précédentes, qui peut être préparé par réaction d'o-phénylènediamine avec de l'acide téréphtalique et de l'acide chlorosulfonique en présence d'acides forts, **caractérisé par le fait que** le temps de réaction est compris entre 10 et 15 heures, l'acide phénylène-bis-benzimidazol-tétrasulfonique obtenu lors de la réaction est, dans une première étape, dissous dans l'eau et est mélangé avec du charbon actif qui est ensuite séparé, et dans lequel le sel disodique de l'acide phénylène-bis-benzimidazol-tétrasulfonique est précipité du filtrat en ajoutant du chlorure de sodium et est séparé et est, dans une seconde étape, à nouveau dissous dans l'eau et dans de la soude caustique et mélangé encore une fois avec du charbon actif qui est ensuite à nouveau séparé, dans lequel, par acidification, du sel disodique pur de l'acide phénylène-bis-benzimidazol-tétrasulfonique se précipite du filtrat, qui est ensuite, le cas échéant, encore épuré.

6. Formulation cosmétique, comprenant un mélange selon l'une quelconque des revendications précédentes.
